# FASCICULE DE BREVET EUROPEEN

(11) **EP 0 418 128 B1**
(45) Date de publication et mention de la délivrance du brevet: **30.06.1993**
(21) Numéro de dépôt: 90402468.4
(22) Date de dépôt: 07.09.1990
(51) Int. Cl.: C02F 1/68, C02F 1/00

(54) **Système de libération continue de la vitamine A dans l'eau d'alimentation**
System zur kontinuierlichen Freisetzung von Vitamin A im Speisewasser
System for continuously delivering vitamin A in feedwater

(30) Priorité: 11.09.1989 FR 8911823
(43) Date de publication de la demande: 20.03.1991
(73) Titulaire: RHONE-POULENC SANTE, 92160 Antony (FR)
(72) Inventeur: Porte, Hugues, F-69300 Caluire (FR); Torres, Ghislaine, F-69006 Lyon (FR)
(74) Mandataire: Le Pennec, Magali

(56) Documents cités:
- EP-A- 0 246 062

## Description

La présente invention concerne un système de libération continue et réguliere de la vitamine A dans l'eau d'alimentation. Elle concerne plus particulièrement un système de libération continue utilisé dans les puits et les forages contenant de l'eau appauvrie en substances organiques et particulièrement en vitamine A.

On estime actuellement à plusieurs dizaines de millions de personnes, la population présentant une déficience en vitamine A. Cette déficience se traduit par des problèmes de xérophtalmie et de non voyance. Les parties du monde les plus touchées sont l'Afrique, l'Asie et l'Amérique Latine. Parmi celles-ci, les zones du Sahel et du Sud-Sahel sont gravement atteintes (Benin, Burkina Faso, Mali et la Mauritanie).

Cette carence se traduit sur le plan de la santé humaine par des xérophtalmies, une résistance affaiblie à l'infection et une mortalité accrue. Une des actions actuellement réalisée par l'Organisation Mondiale de la Santé est la distribution de dose orale de 200 000 unités internationales de vitamine A sous forme de capsule deux fois par an.

Comme il est difficile de pouvoir distribuer biannuellement à chaque habitant la dose nécessaire de vitamine A, on a songé à alimenter chaque être vivant de façon naturelle au moyen de l'eau de boisson. Le seul problème était de trouver un système de libération continue de la vitamine A aussi facile que possible à mettre en oeuvre et permettant une délivrance aussi régulière que possible de la vitamine A sur des périodes prolongées. Le changement du dispositif ne devait pas être réalisé plus de 3 ou 4 fois par an afin d'éviter la présence d'une personne trop fréquemment au niveau de points d'eau particulièrement dispersés dans ces zones désertiques.

La présente invention a permis d'atteindre cet objectif : permettre une libération continue et régulière de la vitamine A dans l'eau de boisson pendant des périodes d'au moins trois mois.

Il existe de nombreux brevets décrivant la libération régulière de principes actifs dont les vitamines dans le corps humain ou animal, comme par exemple dans les brevets US 3 946 734, 3 977 404, EP 40457, EP 262 422 qui ne correspondent en rien au problème qu'essaye de résoudre la présente invention.

Parmi les dispositifs décrivant la libération régulière de principes actifs dans le milieu environnant (eau, air, terre), on peut citer les brevets US 4 300 558, EP 40457 GB 2 182 559, US 4 618 487 qui consistent à établir une différence osmotique entre l'intérieur d'un dispositif qui contient le principe actif à diffuser et un sel minéral et l'extérieur. Le sel en quantité importante attire l'eau du milieu extérieur qui traverse la paroi du dispositif, dissout le sel et le principe actif et s'échappe par un orifice pratiqué dans la paroi du dispositif. La paroi extérieure est généralement perméable à l'eau et imperméable au principe actif.

Ce dispositif présente l'inconvénient d'exiger la manufacture d'un système de délivrance en matière semi-perméable et l'usage d'une quantité importante de sel minéral à côté du principe actif qui sera obligatoirement libéré avec celui-ci. Dans le cas de l'adjonction de principes actifs à l'eau de boisson, nous cherchons à libérer le minimum de sel de façon à ne pas modifier le goût de l'eau puisqu'il s'agit de la faire absorber ensuite à l'homme et à l'animal.

La présente invention a permis de confectionner un dispositif permettant une libération continue et régulière de vitamine A dans les eaux domestiques, notamment sans libération concommittante de sels minéraux, par un système composé d'un récipient en matériau imperméable à l'eau et à la vitamine A ou à ses esters dans lequel est introduit la vitamine A et ou ses esters et muni d'un opercule obturé par une membrane perméable à l'eau et au principe actif présentant de préférence un diamètre moyen de pores supérieur à 0,05 micron et plus préférentiellement compris entre 0,1 et 20 microns.

Le récipient est comme mentionné précédemment imperméable à l'eau et au principe actif. Il est de préférence constitué d'une matière plastique ou de verre et a notamment une forme adaptée à son introduction et à son adaptation au milieu où se trouve l'eau à traiter.

Le principe actif est comme précisé auparavant, de préférence la vitamine A ou un de ses esters tel que par exemple le palmitate. La vitamine A, étant lipophile, sera introduite dans le récipient sous forme hydrodispersible ou hydrosoluble de préférence sous une forme liquide hydrodispersible constituée d'une émulsion huile dans l'eau. Les solides hydrodispersibles de vitamine A vendus sous les marques CRYPTOVIT ou MICROVIT sont aussi utilisables. On préfère utiliser une forme liquide hydrodispersible déjà commerciale vendue sous la marque HYDROVIT titrant 200 000 UI en vitamine A.

La membrane obturant l'opercule sera choisie parmi les matériaux perméables à l'eau et à l'émulsion de la vitamine A. On peut citer au titre des matériaux utilisables :
- les polymères organiques tels que :
   . les polymères acryliques,
   . les polychlorures de vinyle,
   . les esters de cellulose,
   . les polysulfones,
   . les polycarbonates,
   . les polyfluorures de vinylidène,
   . les polytétrafluoroéthylène.
- les polymères inorganiques tels que les membranes céramiques

La dimension de la membrane et le diamètre moyen des pores seront déterminés par l'homme de l'art en fonction du débit de vitamine A désiré. Un flux d'HYDROVIT® compris entre 5 et 50 mg par centimètre carré de membrane et par heure semble suffisant.

Pour permettre ces flux, on peut notamment utiliser des membranes présentant des surfaces de 30 à 100 cm² (ce qui correspond à des opercules de 3 à 6 cm de diamètre) et présentant des diamètres de pores de 1,5 µ à 0,5 µ.

Les dispositifs de l'invention sont introduits de façon immergée dans les endroits contenant l'eau à traiter, surtout les puits et forages et libèrent de façon continue une quantité appropriée de vitamine A à une dose efficace pour assurer le minimum indispensable à l'alimentation humaine et animale sans toutefois atteindre des doses toxiques qui sont estimées aux environ de 600 000 UI par 24 heures et par habitant.

Selon un mode de mise en place, décrit à titre d'exemple, du dispositif selon l'invention, lorsque l'on utilise une émulsion de vitamine A vendue sous la marque HYDROVIT dans un puit débitant 600 litres d'eau par heure et que l'on désire libérer 250 microgrammes par litre de vitamine A pendant une période d'au moins 3 mois. On utilisera environ 5 kg d'Hydrovit® qui seront introduits dans un flacon muni d'une membrane de microfiltration dont les caractéristiques de surface et de dimensions de pores sont adaptés au débit de vitamine A désiré. Ce débit permet l'absorption pour chaque être humain d'une dose de vitamine A journalière de 500 microgrammes sachant qu'un individu absorbe en moyenne 2 litres d'eau par jour.

La présente invention sera plus complètement décrite à l'aide des exemples suivants qui ne doivent pas être considérés comme limitatifs de l'invention.

### EXEMPLE 1

On introduit dans un récipient en verre, de 250 ml, ayant un orifice de 1 cm² de surface, 250 ml d'HYDROVIT® titrant 200.000 UI. L'orifice est ensuite obstrué à l'aide d'une membrane microporeuse dont le diamètre moyen des pores est égal à 1,5 µm (référence : TECHSEP IRIS® 56-25). La membrane est insérée entre deux joints téflon, le tout fixé à l'aide d'un bouchon vissé à la bouteille.

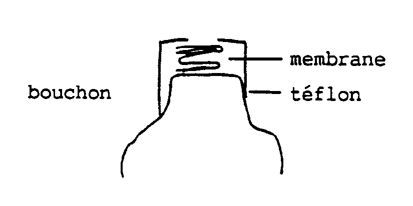

L'ensemble est ensuite immergé dans un récipient contenant 4 litres d'eau distillée maintenue à l'abri de la lumière.

Le milieu d'élution est équipé d'un système d'agitation magnétique animé d'un mouvement lent (100 tr/mn) assurant l'homogénéité de la solution.

### Protocole expérimental de la mesure de cinétique d'élution

Des prélèvements sont réalisés tous les deux jours. La concentration en vitamine A est déterminée par dosage en spectrométrie U.V à la longueur d'onde de 316 nm. Une courbe d'étalonnage préalablement établie avec des solutions étalons de concentration comprise entre 1 et 15 mg/l (en palmitate de vitamine A) permet de connaître la concentration de la solution.

Le milieu d'élution est changé à chaque prélèvement afin d'être dans des conditions "sink".

On entend par condition "sink" des conditions de dilution telles qu'il n'y ait pas de phénomène de couche limite au voisinage de la membrane.

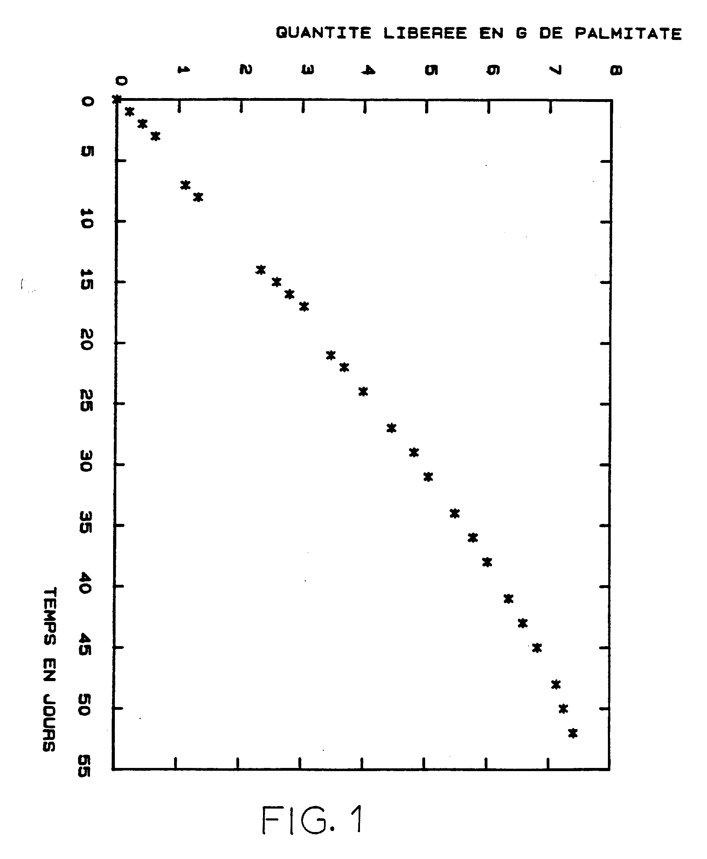

Sur la figure 1 est reportée la courbe correspondant à la cinétique d'élution : quantité de palmitate de vitamine A libérée en fonction du temps

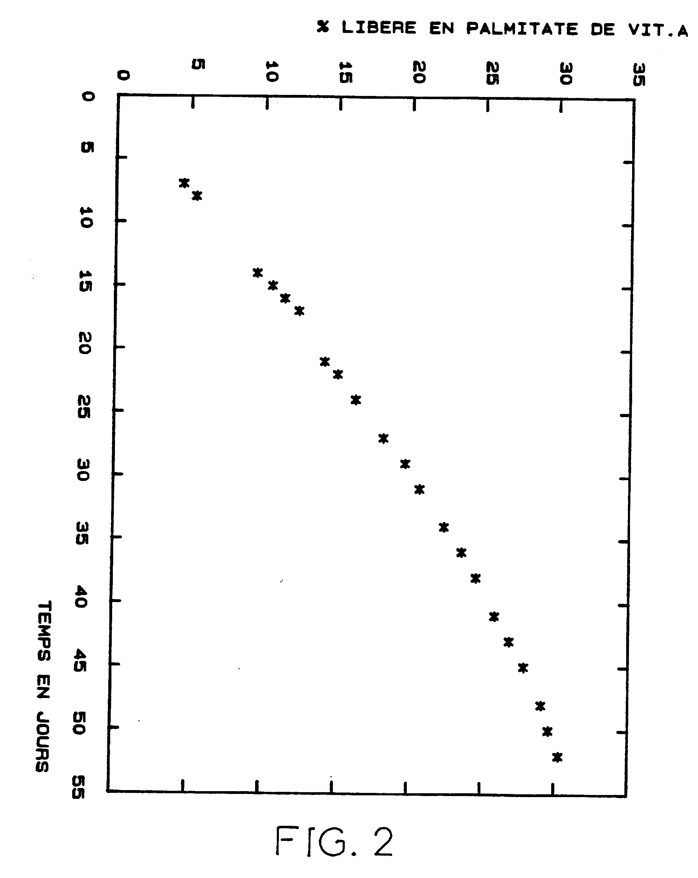

Sur la figure 2 est reportée la courbe de pourcentage de palmitate de vitamine A libérée en fonction du temps pour la même cinétique d'élution.

On peut atteindre sans difficulté, des libérations contrôlées de vitamine A sur des périodes de 3 mois (dans l'exemple 30 % élués en 50 jours) et des flux journaliers correspondant à l'objectif (environ 300 mg/j de palmitate de vitamine A) en utilisant une membrane de 40 cm².

### EXEMPLE 2

On introduit dans un récipient en verre, de 250 ml, ayant un orifice de 1,8 cm² de surface, 250 ml d'HYDROVIT titrant 200.000 UI. L'orifice est ensuite obstrué à l'aide d'une membrane microporeuse dont le diamètre moyen des pores est égal à 0,1 µm (référence : TECHSEP IRIS® 65-02). La membrane est insérée entre deux joints téflon, le tout fixé à l'aide d'un bouchon vissé à la bouteille.

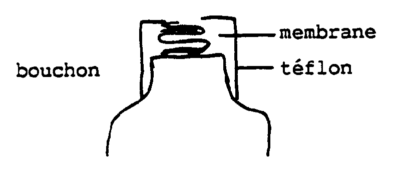

L'ensemble est ensuite immergé dans un récipient contenant 4 litres d'eau distillée maintenue à l'abri de la lumière.

Le milieu d'élution est équipé d'un système d'agitation magnétique animé d'un mouvement lent (100 tr/mn) assurant l'homogénéité de la solution.

### Protocole expérimental de la mesure de cinétique d'élution

Des prélèvements sont réalisés tous les deux jours. La concentration en vitamine A est déterminée par dosage en spectrométrie U.V à la longueur d'onde de 316 nm. Une courbe d'étalonnage préalablement établie avec des solutions étalons de concentration comprise entre 1 et 15 mg/l (en palmitate de vitamine A) permet de connaître la concentration de la solution.

Le milieu d'élution est changé à chaque prélèvement afin d'être dans des conditions "sink".

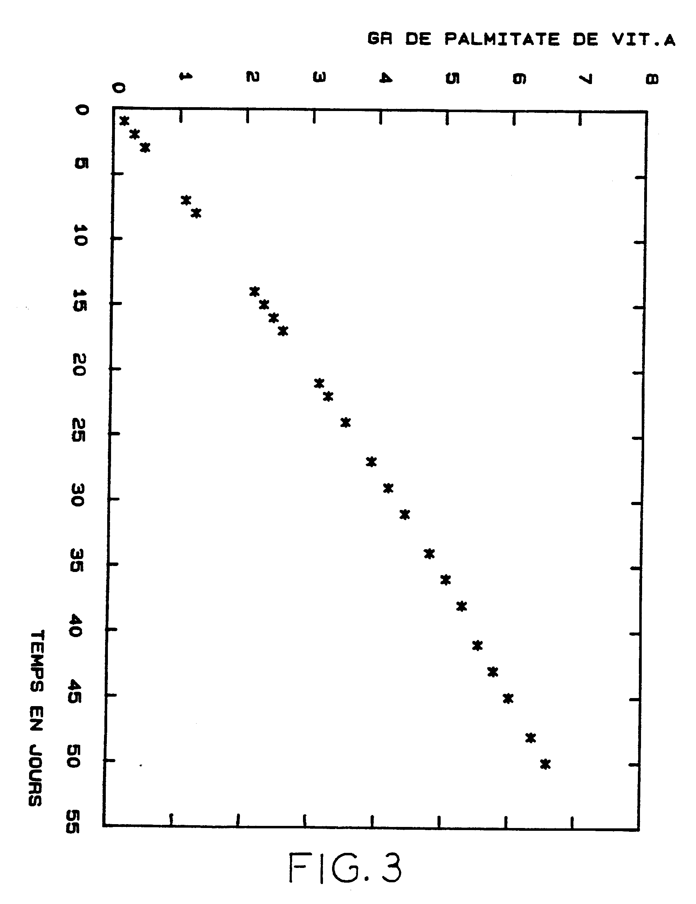

Sur la figure 3 est reportée la courbe correspondant à la cinétique d'élution : quantité de palmitate de vitamine A libérée en fonction du temps.

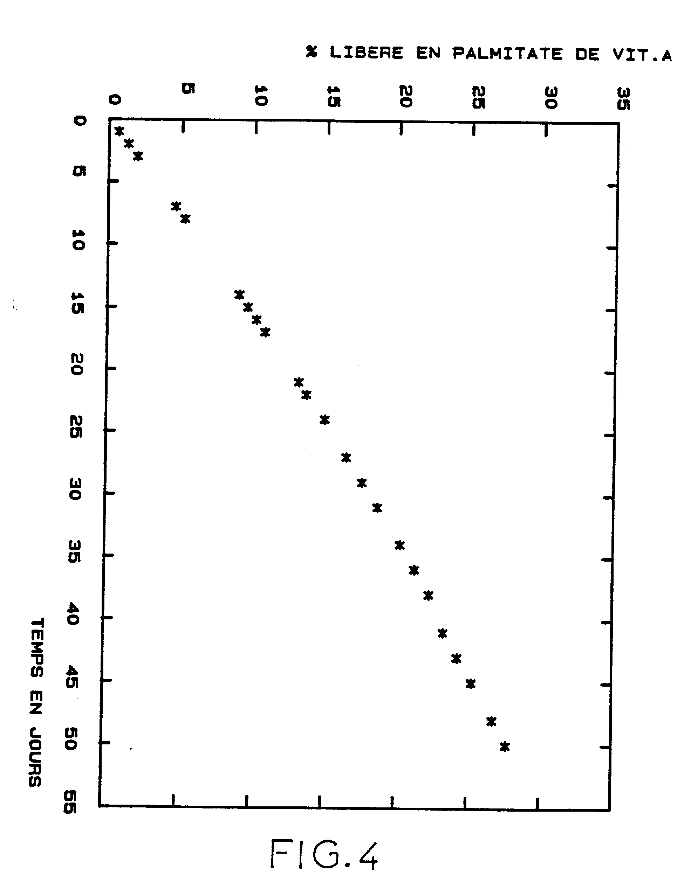

Sur la figure 4 est reportée la courbe de pourcentage de vitamine A libérée en fonction du temps pour la même cinétique d'élution.

On peut atteindre sans difficulté, des libérations contrôlées de vitamine A sur des périodes de 3 mois (dans l'exemple 30 % élués en 50 jours) et des flux journaliers correspondant à l'objectif (environ 300 mg/j en palmitate de vitamine A) en utilisant une membrane de 75 cm².

### EXEMPLE 3

Le système utilisé est le même que dans les deux exemples précédents, la sulface de membrane est de 1 cm².

C'est la nature de la membrane qui a été modifiée, il s'agit d'une membrane en ester cellulosique dont le diamètre moyen de pores et de 0,22 µm (reférence : MF-MILLIPORE/GS).

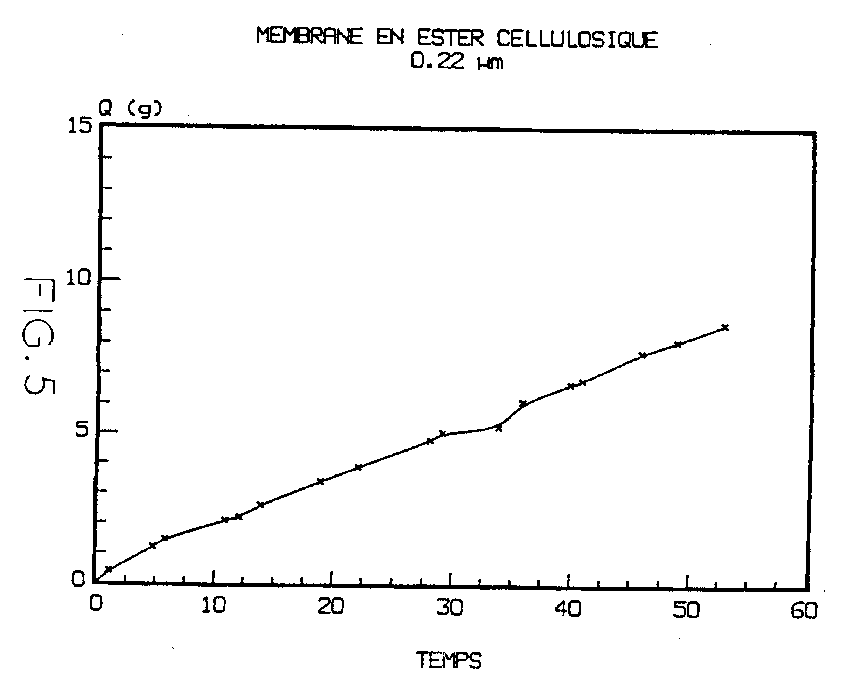

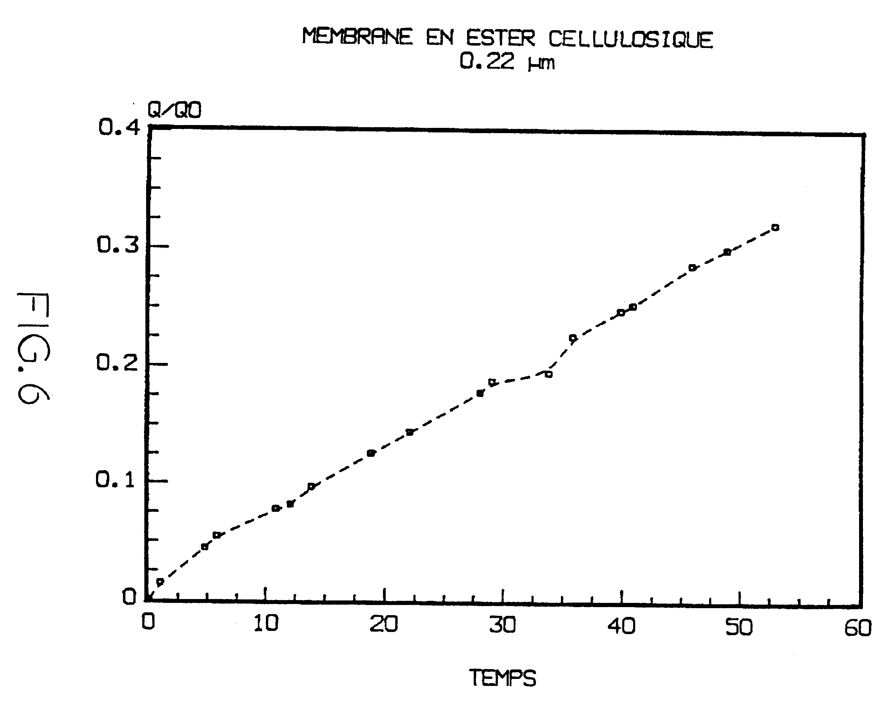

Le protocole expérimental de mesure de la cinétique d'élution est identique, sur les figures 5 et 6 sont reportées respectivement les courbes Q(g) = f(t) et Q/QO = f(t). Les flux journaliers correspondant à l'objectif soit 7,2 g/jour, pourraient être facilement atteints à l'aide d'une membrane de 45 cm².

### EXEMPLE 4

Membrane de nature différente, toujours le même dispositif.

Membrane en PVDF (fluore de polyvinylidène) traitée pour être rendue hydrophile, diamètre de pores moyen = 0,22 µm. Référence : DURAPORE/GV.

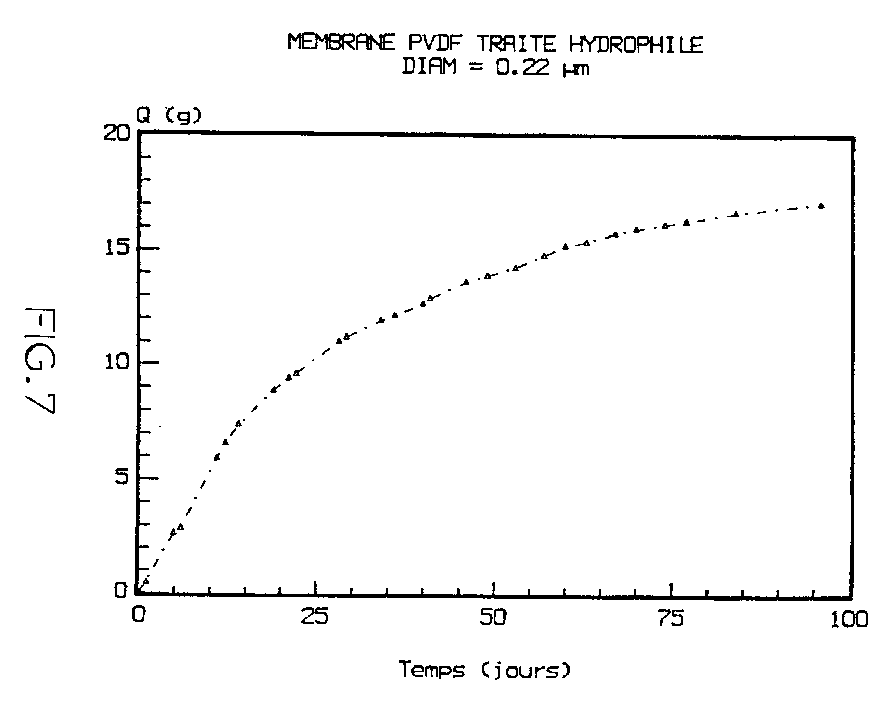

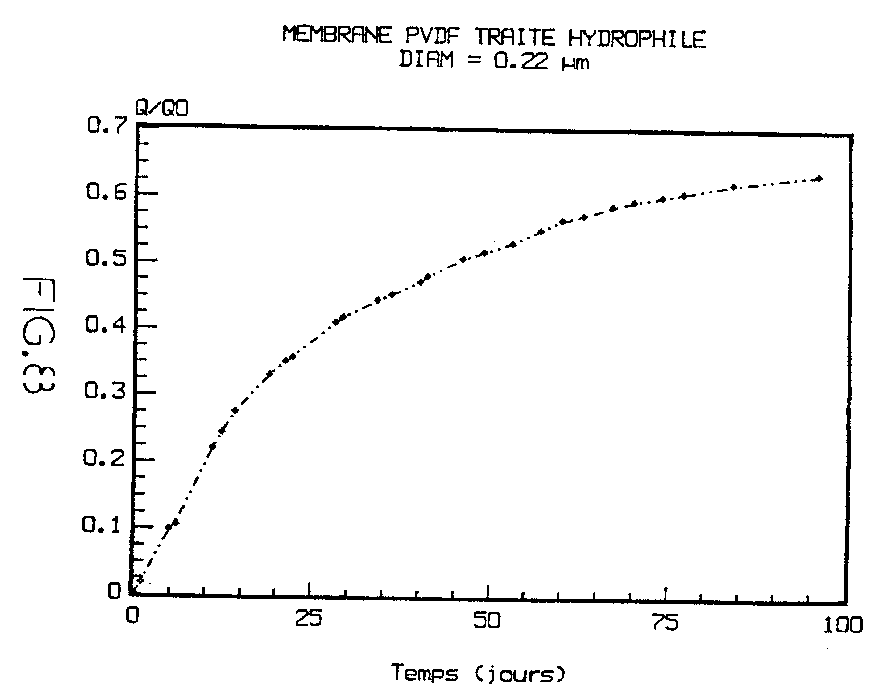

Sur les figures 7 et 8, sont reportées les courbes correspondant aux cinétiques d'élution obtenues, Q = f(t) et Q/QO = f(t).

Un flux journalier de 7,2 g/jour pourrait être atteint en utilisant une membrane d'une surface de 20 cm².

## Revendications

1. Dispositif permettant une libération continue et régulière de vitamine A dans les eaux à usage domestique caractérisé en ce qu'il est constitué par un système composé d'un récipient en matériau imperméable à l'eau et à la vitamine A ou à un de ses esters dans lequel est introduit la vitamine A ou un de ses esters et muni d'un opercule obturé par une membrane perméable à l'eau et au principe actif présentant un diamètre moyen de pores compris entre 0,05 micron et 20 microns.

2. Dispositif selon la revendication 1 caractérisé en ce que le principe actif se présente sous une forme hydrodispersible solide ou liquide.

3. Dispositif selon la revendication 1 caractérisé en ce que la membrane est choisie parmi les polymères ou copolymères organiques tels que les polyacrylates, le polychlorure de vinyle, les esters de cellulose, les polysulfones, les polyfluorures de vinylidène, les polycarbonates, les polytétrafluoroéthylènes ou les polymères inorganiques tels que les membranes céramiques.

4. Utilisation d'un dispositif selon l'une des revendications précédentes pour la libération continue de la vitamine A sans libération concomitante de sels minéraux dans l'eau de consommation domestique.

## Patentansprüche

1. Vorrichtung für eine kontinuierliche und gleichmäßige Abgabe von Vitamin A in Wasser für den häuslichen Gebrauch, dadurch gekennzeichnet, daß sie aus einem System besteht, gebildet aus einem Rezipienten aus einem gegenüber Wasser und dem Vitamin A oder einem seiner Ester undurchlässigen Material, in den das Vitamin A oder einer seiner Ester eingebracht wird, und der versehen ist mit einem Deckel, welcher durch eine gegenüber Wasser und dem Wirkstoff permeable Membran mit einem durchschnittlichen Porendurchmesser zwischen 0,05 Mikron und 20 Mikron abgedichtet ist.

2. Vorrichtung gemäß Anspruch 1, dadurch gekennzeichnet, daß der Wirkstoff in Form eines wasserdispergierbaren Feststoffs oder einer Wasserdispergierbaren Flüssigkeit vorliegt.

3. Vorrichtung gemäß Anspruch 1, dadurch gekennzeichnet, daß die Membran ausgewählt wird unter organischen Polymeren oder Copolymeren wie den Polyacrylaten, dem Polyvinylchlorid, den Celluloseestern, den Polysulfonen, den Polyvinylidenfluoriden, den Polycarbonaten, den Polytetrafluorethylenen oder den anorganischen Polymeren wie den keramischen Membranen.

4. Verwendung einer Vorrichtung gemäß einem der vorhergehenden Ansprüche für die kontinuierliche Abgabe von Vitamin A ohne gleichzeitige Abgabe von Mineralsalzen in Wasser für den häuslichen Verbrauch.

## Claims

1. Device permitting a continuous and steady release of vitamin A into water for household use, characterised in that it consists of a system made up of a receptacle made of material which is impervious to water and to vitamin A or to one of its esters, into which vitamin A or one of its esters is introduced, and provided with a cap closed by a membrane permeable to water and to the active substance which has a mean pore diameter of between 0.05 micron and 20 microns.

2. Device according to Claim 1, characterised in that the active substance is in a solid or liquid, water-dispersible form.

3. Device according to Claim 1, characterised in that the membrane is chosen from organic polymers or copolymers such as polyacrylates, polyvinyl chloride, cellulose esters, polysulphones, polyvinylidene fluorides, polycarbonates, polytetrafluoroethylenes or inorganic polymers such as ceramic membranes.

4. Use of a device according to one of the preceding claims for the continuous release of vitamin A without accompanying release of inorganic salts into water for household consumption.
